# EUROPEAN PATENT APPLICATION

(11) **EP 1 298 433 A1**
(43) Date of publication of application: **02.04.2003**
(21) Application number: 01123435.8
(22) Date of filing: 28.09.2001
(51) Int. Cl.: G01N 33/50, G01N 33/543, B01J 19/00, G01N 33/52

(54) **Device and method for the generation of arrays of substance mixtures by diffusion**

(71) Applicant: EMC microcollections GmbH, 72070 Tübingen (DE)
(72) Inventor: Brock, Roland Dr., 72074 Tübingen (DE)
(74) Representative: Mütschele, Thomas

(57) **Abstract**

The invention relates to a novel device for efficiently generating a large number of different substance mixtures of at least two substances and assaying the interaction, especially the joint interaction, of at least two substances with at least one target, as well as to a method for assaying the afore-mentioned interaction and the use of the method and the device for assaying substance(s)-target(s) interaction(s).

## Description

The invention relates to a device for efficiently generating a large number of different substance mixtures of at least two substances and assaying the interaction, especially the joint interaction, of at least two substances with at least one target. Furthermore, the invention relates to a method for assaying the above-mentioned interaction and also to the use of said device and method.

Currently, biological assays aim at identifying from a multitude of compounds, e.g. test substances, single compounds that exert a desired action, e.g. acting as enzyme inhibitors. These compounds may be derived from all commonly used and known sources. Assays are carried out with a single compound per test. The biological activity in terms of the concentration required to exert a half-maximal effect is determined by serial dilution of the test compound into a multitude of separate test containers, mostly in the form of wells in microtiter plates of various formats. Each dilution requires a working step either by the experimentator or by a robotical device. The assay for all compounds and their dilutions starts after preparation of all dilution series, either by addition of the target with respect to which the compounds are tested, of an additional compound required to start the reaction or by simultaneous transfer of all samples to the test targets. If timing is critical the starting points of subsets of tests can be delayed by defined intervals that will usually depend on the time required to handle each subset in parallel.

In a modification of the above-mentioned test scheme, each test in an assay contains a mixture of compounds (usually equimolar mixtures for all compounds present in a test). These mixtures can either be the outcome of chemical reaction steps carried out in one reaction vessel (e.g. peptide libraries with randomised positions) or generated post-synthesis through mixing of single substances. In the current state of the art dilution series are performed for the whole mixture of compounds, such that the relative concentrations of the compounds in all tests of a dilution series are kept constant. If such a mixture of active compounds exerts the desired biological activity, the complexity of the mixture in terms of the number of different compounds present in the test is successively reduced by protocols referred to as deconvolution until (a) single active compound(s) is (are) identified. A hit defines such a single compound exerting the desired biological action.

This means, that the present assay technologies are limited in that only single active compounds are identified for one desired biological effect. Even though mixtures of compounds may be more active at lower doses than any single compound alone, or a compound may only be active in the presence of another compound, the identification of optimal combinations of compounds is not implemented in drug development in a systematic and efficient way.

In the present art, in the field of therapy development, physicians combine drugs as part of the medical therapy. One example is the combination of cytostatika in the treatment of cancer, where the optimal selection of a combination is vital for optimal success of chemotherapy. For instance in the so-called cloned tumor-cell assay, tumor cells are cultivated in the cell culture, and before therapy different cytostatika combinations are tested. Nevertheless, only a limited number of cytostatika combinations and concentrations of each compound can be tested, being a risk of missing the optimal combination or imposing a too high load of cytostatika on the patient.

Analogous to the identification of optimal drug combinations, chemical reactions as well critically depend on the chemical milieu (e.g. temperature, pH, solvent) as well as the absolute and relative concentrations of the reactants. For a certain class of organic chemical reactions - multi-component-reactions on a solid support - a number of reactants are present in the reaction mixture simultaneously and the product is formed through several reaction steps without isolation and purification of reaction intermediates. In the same way as for the testing of different combinations of potential drugs, an optimisation of reaction conditions requires tedious preparation of a large series of separate reaction mixtures either by a robotic system or the experimentator.

An important reason for this limitation is the lack of methods and devices to efficiently perform assays in which the absolute and relative concentrations for test compounds are varied for a large number of testings. It is the purpose of the inventive device and method to overcome this limitation by using diffusion for a superposition of concentration gradients of at least two substances thereby efficiently generating large numbers of substance mixtures of which the concentrations of at least two substances vary at any place of the device where testings are performed and assaying the interaction, especially the joint interaction, of at least two substances with at least one target.

According to the prior art, and in contrast to the present invention, diffusion and other passive spreading mechanisms such as capillary forces have been used for a uniform distribution of substances. Implementations of either one of these spreading principles have been described with respect to diagnostic test systems, primarily test stripes as can be found for example in US 3,992,158, US 4,042,355, US 4,144,306, US 5,547,833.

Another field of inventions in which diffusion has been employed as a transport process relates to so called free-format assays in which targets on which a collection of compounds are to be tested are incorporated in a matrix and the test substances are delivered to the matrix by means such as pipetting or contact of a layer carrying the substance collection at defined position with the matrix incorporating the target. The substances enter the matrix by means of diffusion. The matrix may be present in a dry form and the compounds are applied in solution in the form of drops. The term "free-format" refers to the fact that the relative positions of the test sites are not determined by a physical separation of test sites as for example wells in a microtiter plate.

In US 5,076,813, for example, such a "free-format" test format is described. Test substances have to be placed far enough apart from each other so that no mixing due to lateral diffusion occurs. If an activity is detected one must be able to assign it to a single active substance based on the position of the assay device where the activity is present.

In the same way as in US 5,976,813 other prior art employing diffusion for transport of substances has sought to avoid diffusive mixing of test substance. One example is the testing of substances which are released from beads and transferred into an agarose layer in which cells are imbedded (Proc. Natl. Acad. Sci. (USA), Vol 91, pp. 1614-1618,1994). This more recent development is another example of gel assays for antibacterial or anticancer agent, and are also similar to the familiar immunological assays in which an antigen/antibody interaction is measured in a gel.

Another family of inventions employs diffusive transport to test the permeation of substances across a semipermeable membrane, carrying a monolayer of tissue culture cells. One example of such device is given by WO 99/21958. Nevertheless, in this international patent application all test sites have to be physically separated from each other. Diffusion occurs only across the semipermeable membrane, from a single well on one side of the semipermeable membrane to the corresponding well on the other side of the semipermeable membrane.

In contrast to the present art, the inventors have realised that the generation of substance mixtures by diffusion may serve to overcome the above-mentioned limitations in assays for biologically active compounds, therapy development and diagnostics as well as optimisation of reaction conditions in chemical syntheses. The object of the invention is to provide novel devices and methods enabling the efficient generation and testing of substance mixtures in which the concentrations of at least two substances vary at any place of the device where testings are conducted, so that the above-mentioned and other disadvantages are overcome.

This limitation of the present art is solved by the subject matter of independent claims 1, 22, 28 and 29. Preferred embodiments are given in the dependent claims 2 to 21, 23 to 27 and 30. The wording of all claims is hereby incorporated into the content of the description by reference.

Accordingly, one aspect of the present invention is to provide a device for assaying the interaction, especially the joint interaction, of at least two substances with at least one target, in which mixtures of said substances are generated, comprising a diffusion medium for generating mixtures of said substances by diffusion, and at least one discrete site for immobilizing at least one target with respect to the diffusion medium. In general, this diffusion medium can be any medium capable of generating mixtures of substances by diffusion. Preferably the diffusion medium is a diffusion layer having a considerably greater extension with respect to two of its three dimensions compared to the extension (thickness) of its third dimension. By using diffusion as the means for generating substance mixtures, for which the concentrations of at least two substances vary at each site where a testing is performed, it is possible with the inventive device to increase dramatically the conditions that can be tested with a limited number of manipulations. It is a characteristic of the present invention that in contrast to the present art, the number of manipulations does not scale with the number of testings performed. By increasing the size of the device or the density of sites where testings are performed, the number of testings scales with the size or the density. However, the number of manipulations to apply test substances to the device remains the same.

In the diffusion medium, linear concentration gradients will be established, with highest concentrations at the application site and lowest concentrations at the sites distal from the application site. These concentration gradients will change in a time-dependent manner. The temporal evolution of the concentration gradients will be determined by the diffusion constants of the substances in the respective diffusion medium.

According to the invention (by a non-restrictive definition), preferably a substance can be any molecule which is assumed to either a) exert an action on a target, b) modify the response of a target to another substance, c) modify the action of a substance on a target, d) combine with another substance to a whole, and especially with respect to the use of the device and methods in the optimisation of chemical reactions e) catalyze a chemical reaction, f) control a chemical reaction, g) be part of a chemical reaction, or a combination thereof. Different substances within one assay can either belong to one class or to a variety of classes. Realisations are also considered in which a substance can be assigned. to a class only after the assay has been carried out (e.g. as acting on a target or influencing another substance action on a target by promoting/controlling/being part of a chemical reaction).

For purposes of a (non-restrictive) definition, according to the invention a target is an entity on which a substance or a combination thereof acts or interacts with. Targets can be preferably
a) purified natural molecules from natural sources or recombinant techniques, especially proteins and nucleic acids, b) prokaryotic and eukaryotic cells, and c) substrates of any kind on which the substances participate in, catalyze or influence chemical reactions. One target can contain targets of lesser complexity, e.g. an eukaryotic cell contains a large number of structurally and functionally different molecules that may function as targets with respect to the intention of the assay.

In a preferred embodiment, a device is provided in which the lateral diffusion in two dimensions is decisive for the generation of said mixtures of substances in the diffusion medium.

In another embodiment according to the invention, the diffusion medium is a three-dimensional diffusion medium in which the generation of said mixtures of substances takes place in all three dimensions. This diffusion medium can be e.g. in the form of a cube or other suitable geometry.

In another aspect, the inventive device is characterized in that the discrete sites in which at least one target is immobilized are part of or are formed by the diffusion medium. If said discrete sites are not part of or are not formed by the diffusion medium, they are preferably part of or are formed by a(n) (separate) assay medium, especially an assay layer. This assay medium can reversibly be connected with the device, especially with the diffusion medium for establishing the contact between target(s) and substance mixture(s) necessary for transfer of the substance mixtures to the assay medium.

With all inventive embodiments the discrete sites can be in the form of cavities (assay cavities), preferably formed in said assay layer.

The term "assay cavities" is used as the general term referring either to physical cavities in the common sense or to places on the device where a read-out is performed. Assay cavities can be generated by e.g. physicochemical structuring which is exemplified but not limited to a) hydrophobic ridges separating hydrophilic assay cavities optionally microstructured (as indicated in the next point b)), and b) ridges microstructured with assay solution-repellent microstructures. The backside of the assay medium/assay layer may also be coated with assay solution-repellent chemical coating or microstructured with assay solution-repellent microstructure. The latter embodiment is used preferably when contact of the assay medium/assay layer with the diffusion medium/diffusion layer is only transient.

In another aspect of the invention, the discrete sites are arranged in a preferably regular pattern, wherein preferably said discrete sites are for example equally or logarithmically spaced for probing linear or logarithmic dilution patterns with respect to the concentration gradients of substances in the diffusion medium. This will be discussed later with reference to the drawings.

In a further preferred embodiment the discrete sites are separated from the diffusion medium, preferably by a separation medium, especially a separation layer. The separation layer can be formed by a semipermeable membrane, wherein preferably said semipermeable membrane is permeable to at least one substance of the substance mixture and substantially impermeable to the targets. In other embodiments the separation layer can functionally be replaced by covalent or non-covalent coupling of the targets to an assay medium in which the discrete sites are also preferably formed as cavities, or by covalent or non-covalent coupling of said targets to microspheres located inside said cavities.

The term "semipermeable" is used according to the invention in that it obstructs assay targets from leaving the assay medium and entering into the diffusion medium, while letting sample (substance) components through. In corresponding embodiments the separation medium/layer is obstructing lateral diffusion of solutions and allowing vertical diffusion only. Either by combination of assay medium and separation medium or by integration of both media in one, vertical diffusion of sample (substance/substance mixtures) from the diffusion medium into the assay cavities is only possible at the location of assay cavities. Depending on the chosen type of assay the material of the separation medium/layer will be selected by the person skilled in the art. For instance in a biological assay the separation medium/layer may be coated with chemicals that guarantee biocompatibility. This realisation may be preferable if both assay medium and separation medium are both integrated into one, e.g. by wet-etching techniques from silicon wafers. If a combination of assay medium and diffusion medium already fulfills the requirements of semipermeability and biocompatibility then the separation medium/layer may be omitted.

If immobilization of the target is possible in a way that no diffusion of targets in the assay medium occurs while the assay is performed, the functions of the assay medium and diffusion medium may be combined in one single medium, preferably one single layer. In this embodiment, read-outs may be performed for a continuous gradient of assay conditions.

In another preferred embodiment, the device further comprises at least one substance application site for bringing at least two substances (samples) to the assay device and preferably for establishing the necessary contact between sample and diffusion medium. These substance application sites can be part of or formed by a sample medium, especially a sample application layer. The substance application sites can be for example in the form of a fiber or resin-filled hole, a fiber or resin pad, a cavity, a dried substance spot on a solid support and/or a membrane with little (lateral) diffusion. However, any other material with little non-specific interaction with the samples (substances) and form known by the person skilled in the art can be chosen. Alternatively, the sample medium can be connected to or substituted by a microfluidic device comprising ducts or channels connected to inlets and outlets in the diffusion medium.

The sample medium locally delivers sample (substance) to distinct locations of the diffusion medium. The sample medium can be made from any membrane or other suitable material that can be brought into contact with the diffusion medium without causing sample (substance) mixing by processes other than diffusion. The substances can be placed on the sample medium in any pattern that is technically compatible with the realisation chosen for the sample medium. As a consequence any kind of overlapping concentration gradients can be formed. These are exemplified but not limited to gradients in which substances diffuse against one another and gradients in which substances diffuse along with one another.

In specific embodiments the sample medium can be integrated into the assay medium in form of separate microcavities (microcavities here must be understood in the same broadened sense as assay cavity). Substance e.g. can either be soaked into small patches of fiber or resin material present in cavities in the assay medium that - when working the assay - are in contact with the diffusion medium, or dried onto the backside of the assay medium. In another aspect, sample medium and diffusion medium can be one joint medium. Substance solutions are spotted locally onto the medium. With this realisation it is advantageous that the sample medium is stored in a dry form or that the substances are linked covalently to the sample medium and released upon beginning of the assay.

In another preferred embodiment the device comprises further a solid support, preferably for improving the mechanical stability of the assay.

According to the invention, there are especially preferred embodiments, in which the device comprises the following sequence of components, namely a solid support, a sample application layer (comprising substance application sites) a diffusion layer, a separation layer, and an assay layer (comprising discrete sites at which target id immobilized).

In general, each layer can be integrated into each other layer, e.g. sample application layer and assay layer can be integrated into each other or the sample application layer and diffusion layer, respectively.

In an especially preferred embodiment, a device comprising at least two of the devices as described above is claimed. This allows the working of at least two assays in parallel.

In another aspect of the invention, a method (in which mixtures of said substances are generated) for assaying the interaction, especially the joint interaction, of at least two substances with at least one target, is claimed. This method is characterized in that said mixtures are generated by diffusion of said substances in a diffusion medium, especially a diffusion layer, that interaction of said mixtures takes place with targets substantially immobilized with respect to the diffusion medium at discrete sites, said interaction is detected preferably at a point in time, when the concentrations of at least two substances vary at each site where a testing is performed, and the concentrations of the substances are derived from the positions of the device where the interaction is detected.

The inventive devices and methods can be used to test different targets in relation to one combination of substances and/or to test the same targets in relation to different combinations of substances. Furthermore, the invention can be used to supply data for establishing models describing the behaviour of a target in response to the combinations of substances in a predictive fashion.

The efficient identification of combinations of substances for which the combination may potentate the desired biological aspect of each individual compound or for which the biological effect may only be present for the combination of compounds is a preferred field of application.

This includes the identification of drug combinations as an integral part of the drug development process. By the inventive method, slightest variations of relative and absolute concentrations of compound mixtures can be assayed. Depending on the realisation of the method, all samples (substances) can be potential drug candidates, which are supposed to address either only one target, or which are supposed to address more than one biological target either with distinct or overlapping specificities for all drug candidates or subsets of these. These embodiments of the invention are exemplified but not limited to the identification and improvement of optimum drug combinations in cancer therapy by efficient screening of a large number of combinations and concentrations of e.g. cytostatika with a minimum of tumor material from the donor by automated high-throughput read-out technology. For instance kits may be provided with which a physician can test a large number of conditions in a standardized way and which are characterized by the fact that with a limited number of manipulations a greater number of conditions can be tested. The number of conditions only depends on the ratio of the diffusion area and the assay cavities per area. Combinations of antibiotics may be tested in a very similar fashion.

Alternatively, at least one sample (substance) is not a drug candidate but meant to vary the chemical environment, undergo a chemical reaction with at least one of the drug candidates, or vary the pharmacokinetics or pharmacodynamics or a combination of both of the drug candidates. Additionally, one of the samples (substances) not being a drug candidate may alter the response characteristics of at least one of the targets. If the targets are living cells, at least one of the samples (substances) not being a drug candidate may change the behaviour of living cells. These realisations encompass but are not limited to substances that induce cancer, apoptosis, necrosis, cell proliferation, cell differentiation, cell migration, the response of T-lymphocytes to target cells, micropinocytosis, endocytosis, phagocytosis, antigen-processing.

One example for the second class of applications is the assaying of the effect of substances on the activation of T-lymphocytes carried out in the presence of varying concentrations of a cytokine that alters the response characteristic of a T-lymphocyte to a stimulus.

Other applications relate to regenerative biology, which intend to reproduce the spatial and temporal pattern of cellular proliferation and differentiation, ex vivo or in vivo. In the induction of differentiation, substance gradients and the absolute and relative concentrations of differentiation factors are decisive. The analysis and artificial induction of such regulatory processes and thereby tissue differentiation require methods for the efficient generation of mixtures of differentiation factors and testing of large numbers of different conditions with only gradual differences in the relative and absolute concentrations of substances.

Other applications relate to the identification of substance mixtures exerting a biological activity as is the case in the field commonly known as herbal medicine. In these applications, each sample (substance) corresponds to a fraction (either one substance or by itself a group of substances) of all substances isolated from natural sources, preferably plants that in an unknown combination with respect to constituents and relative quantities constitute the active principle. The assay device and method serve to identify the optimal recombination of substances constituting the active principle.

Another field of application may be the so-called solid phase organic chemistry. With the inventive device and method a large number of reaction mixtures can be formed automatically with a limited number of manipulation steps for the preparation of the synthesis that is independent of the number of reaction mixtures to be tested.

The read out of the interaction of the substance(s) with the target is exemplified but not limited to fluorescence, mass spectrometry, high performance liquid chromatography, reverse PCR, capillary electrophoresis and/or changes of the electric properties of any assay constituent, preferably addressed by one or more microelectrodes. Also any other method of measuring the interaction of the substances with the targets is possible and claimed in accordance with the invention. The above-mentioned methods can be further characterized by any feature of the device as defined above.

The methods and the device as defined above can be used to investigate the interaction of substances to targets like e.g. prokaryotes, eukaryotic cells, cellular organelles, biomolecules from natural or synthetic source and/or synthetic molecules with no correspondence in nature. However, also other targets known by the person skilled in the art can be investigated. For instance it is possible to test combinations of anticancer drugs for optimisation of said drugs in anticancer therapy or to test combinations of antibiotics.

The assay can be performed by different modes of operation. In one mode of operation, the targets are present in the assay cavities and the test substance is added to or released from the substance application sites. The assay may be read out at any point in time or different time points in a time-resolved manner.

According to the present invention the interaction of the targets with said substance mixtures is analysed at a point in time, when for at least two substances the equilibrium with respect to the distribution in the diffusion medium has not been reached. In this case, the concentrations of at least two substances varies for each position where a testing is performed.

Alternatively, because of a spatio-temporal evolution of substance mixtures that varies for each location where a testing is performed, the response of a target may vary for each position of the assay device, even if the read-out is performed at a point in time, when equilibrium with respect to diffusion of the substances has been reached. For example, targets closer to the substance application site may have been influenced stronger, due to longer exposure to higher concentrations of the substance.

In another mode of operation the targets are not present in the assay cavities at the beginning of the assay. Only when the substance gradients have been formed the targets are applied to all assay cavities at the same time. This operation can be performed by a nanopipetting device or preferably by spraying the target in the form of small droplets into the assay cavities. In this realisation, the targets are not exposed to a temporal evolution of substance gradients but get in contact with the substance mixtures, once the concentration gradients for which the read out shall be performed, have been formed.

One specific embodiment of the assay aims at preserving a gradient at a specific point in time. This may be achieved by providing assay layer and samples (substances) with chemical functionalities that only react with each other upon activation or upon change of the chemical conditions. The initiation of a chemical coupling reaction upon activation can be realised by means of so called caging groups as described for example in Methods in Enzymology, Vol. 291. In one realisation, the assay layer is functionalised with caged sulfhydryl groups whereas the samples (substances) are modified with maleimido reactive groups. As a caging group a 2-nitrobenzyl-moiety may be employed that is cleavable by light. Once the caging group is released by illumination, the sulfhydryl groups will react with the maleimido groups and thereby immobilize the substance and preserve the gradient present at the point in time of illumination. Instead of using caged sulfhydryl groups and maleimido groups any other appropriate combination of caged functionality and reactive group such as caged amino groups and epoxy groups or maleimido groups may be employed. It is also possible to derivatize the samples (substances) with the caged functionalities and derivatize the assay layer with the reactive groups. The initiation of a coupling reaction by a change of chemical conditions is exemplified by derivatizing the assay layer with amino-groups and the samples (substances) with epoxy groups. The gradient is formed at chemical conditions at which no reaction takes place. For the pair of an amino-group functionality and an epoxy-reactive group this may be achieved by adjusting the pH of the solution to below 6. Upon a sudden increase of the pH to above 8, the coupling reaction will take place. Such a change of the chemical conditions without perturbance of the gradient may be achieved by spraying a solution with the appropriate pH onto the assay layer.

In a further modification of this embodiment an additional functionality (that permits a controlled release of the samples (substances) after immobilization) is provided either as a linker between the assay layer and said reactive group or between the functionality of the sample (substance) reacting with said reactive group and the part of the sample (substance) exerting the activity with respect to the intention of the testing. Such a functionality is exemplified but not limited to an enzymatic cleavage site. If a process other than light was employed for the immobilization of the substance the release of the samples (substances) may occur by light using a photocleavable linker. For this specific embodiment in which the samples (substances) may be released, the localization of the reactive groups for immobilization is not restricted to the assay layer but may instead or additionally also take place in the diffusion layer. The embodiments, in which the gradients are presented may preferably be combined with the embodiment in which the targets are added once the gradients have been formed.

In another mode of operation, the substance mixtures are formed in a diffusion medium/layer, and the targets are in the assay cavities. However, during the formation of the substance gradients, the assay medium/layer is not in contact with the diffusion medium/layer. The substance mixtures are transferred into the assay cavities by establishing a transient contact of the assay medium/layer with the diffusion medium/layer. In this mode of operation, the diffusion medium/layer can be used to sequentially transfer the concentration gradient into a large number of different assay media/layers. In this realisation, the targets are not exposed to a temporal evolution of substance gradients but get in contact with the substance mixtures, once the concentration gradients for which the read out shall be performed, have been formed.

For the specific embodiments in which the samples (substances) are immobilized by reaction with reactive groups, targets may be added either once the samples (substances) have been immobilized or directly after release of the samples (substances). Due to the preservation of the gradients by immobilization, in these specific embodiments the assay devices may be stored and/or transported with preformed sample (substance) gradients.

The concentrations of substances at a specific position of the device where an activity is detected can be derived based on the knowledge of the volume of the device that the substances can enter, the position of each substance application site relative to the position of the read-out, the amount of substance applied to the respective substance application site, the diffusion constant of the substance in the diffusion medium and the time over which diffusion took place. Alternatively, a reference substance, that can be detected for example by fluorescence and does neither interact with the target nor with the substances, can be applied together with the test substance as a reporter to the same substance application site. A person skilled in the art may devise a way for deriving the concentration of the test substance from the signal detected from the reporter. For example, the test substance and reporter substance may be added in amounts for which the ratio is known. If the signal detected for the reporter is calibrated for the determination of concentrations, then the concentration of the test substances may be derived directly from the reporter signal. For each test substances a different reporter substance will preferably used, so that the reporter signals do not interfere with each other.

Other modes of operation compatible with the general working principle of the device and method may be possible. It is understood that diffusion and interaction of the samples (substances) with the targets can only take place, if the diffusion medium is soaked with an appropriate solution and the assay cavities are filled with this solution. The solution in the diffusion medium and the assay layer may differ from one another according to the characteristics of the separation layer. For biological assays the solution will be an aqueous buffer compatible with the structural and functional integrity of the targets. For applications of the assay device and methods with respect to the optimisation of chemical reaction conditions this solution will be the appropriate aqueous buffer or organic solvent.

The described features and further features of the invention can be gathered from the following description of preferred embodiments in conjunction with the subclaims. The individual features can be implemented separately or in the form of subcombinations.

In the drawings it is shown:
- Figure 1:: Side-view of an assay device
- Figure 2:: Top-views and side-views of an assay device and working principle
- Figure 3:: Side-views of different realisations of assay cavities
- Figure 4:: Top-views of assay devices for testing different dilution patterns
- Figure 5:: Top-views and side-views of several options for the design of the sample layer

- Figure 6:: Top-views and side-views of several options for the design of the diffusion layer
- Figure 7:: Top-views and side-views of several options for the design of the separation layer
- Figure 8:: Top-view of an assay device consisting out of 4 x 4 single assay devices
- Figure 9:: Diffusion driven formation of inverse diagonal substance gradients on silicium nanotiter plates
- Figure 10:: Gradient formation in cellular applications
- Figure 11:: Enlargement of assay cavities of figure 10

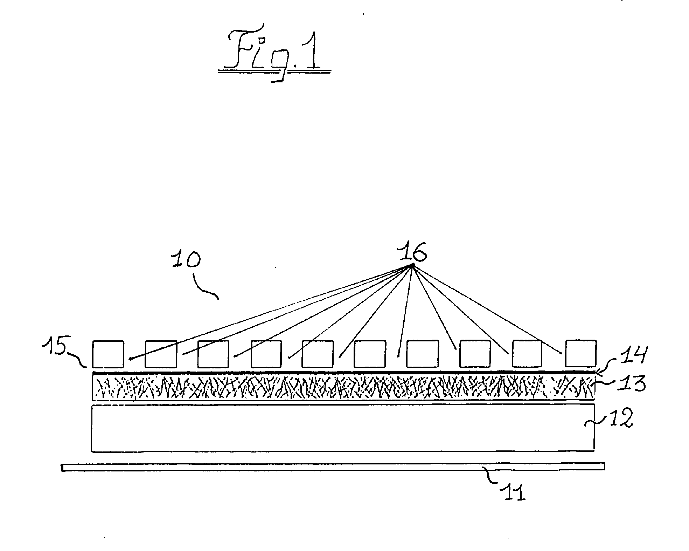

In figure 1 a general schematic of one possible realisation of an assay device (10) is shown comprising a solid support (11), preferably for improving the mechanical stability of the assay, a sample application layer (12) which may comprise substance application sites for bringing the sample (substance) to the assay device, and especially for establishing the contact between sample and diffusion layer, a diffusion layer (13) where the generation of mixtures of substances by diffusion takes place, a separation layer (14) for separating the diffusion layer from the assay layer, and an assay layer (15) which may comprise target immobilizing sites for immobilizing the target, for instance assay cavities (16). In general, all components of said device could reversibly be connected with the other components of the device and/or functionally be replaced by or integrated with any other component, depending on the chosen type of assay. To perform an assay the diffusion layer will be soaked with a solution appropriate for the respective testing and assay cavities will be filled with the solution so as to probe the interaction of the substances with targets.

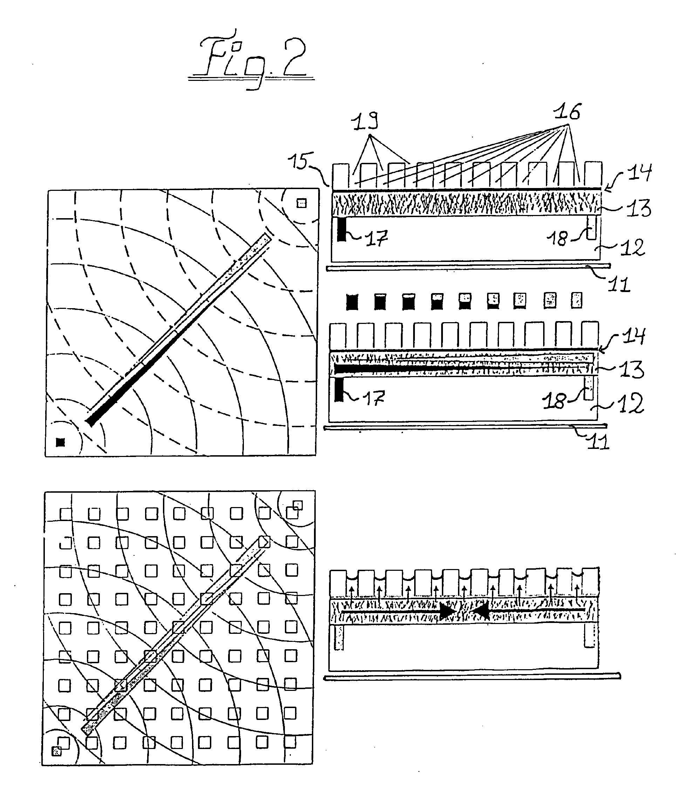

In figure 2 the working principle of one possible realisation of the inventive device is illustrated. The components of the device are the same as described in figure 1. The samples (substances) are marked in black and grey. As can be seen on the upper right side, concentration gradients for sample (substance) A (17, black) and sample (substance) B (18, grey) are built-up by lateral diffusion in the diffusion layer, so that in each assay cavity (16) the sample (substance) concentration represents the underlying concentration gradient. The substance mixtures are transferred into the assay layer (15), wherein the targets (19) are immobilized. By this way any kind of overlapping concentration gradients can be formed. These are exemplified but not limited to gradients in which substances diffuse against one another and gradients in which substances diffuse along with one another. The number of different concentrations only depends on the ratio of the diffusion area and the assay cavities per area as is illustrated in figure 2, showing that each assay cavity has different relative concentrations of the samples (substances). In addition, in figure 2 the filling of the assay cavities with an appropriate solution - the same solution with which the diffusion medium is soaked - and the working principle for diffusion and interaction of the samples (substances) with the targets is illustrated.

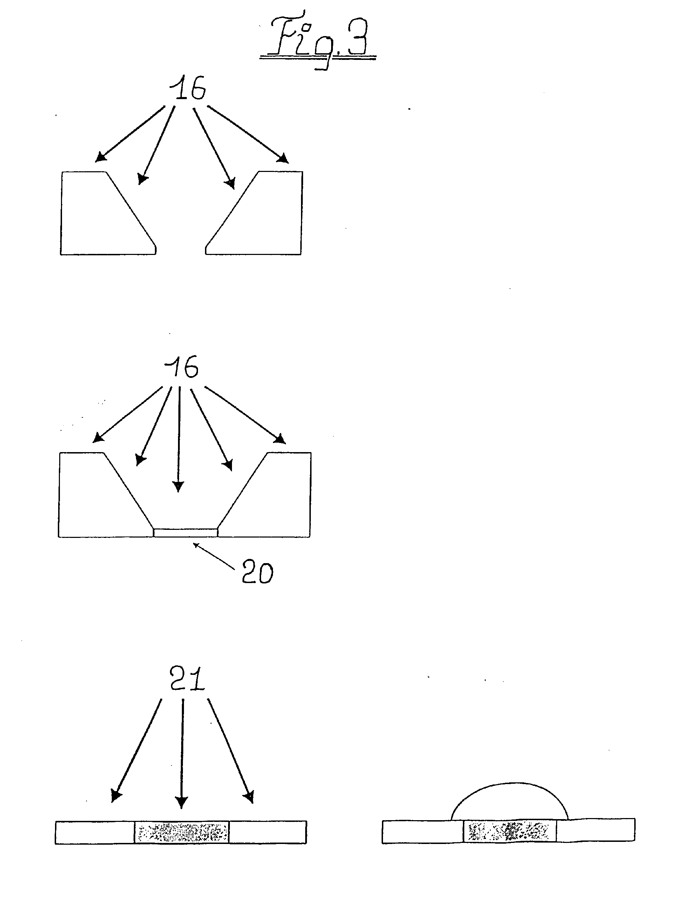

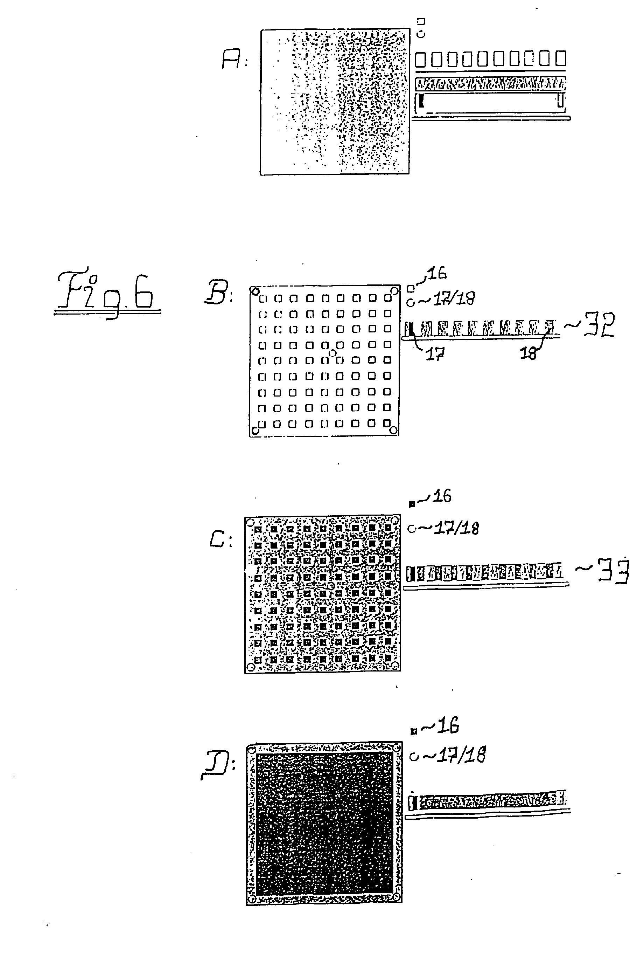

In figure 3 different possible options for the design of the assay cavities (16) are shown. The shape of each assay cavity may be determined by the manufacturing process as well as by the characteristics of the used material. The assay cavities can be separated from the surrounding components of the device by a(n) (integrated) separation layer, for instance a semipermeable membrane (20) as is illustrated in the middle of figure 3. Said semipermeable membrane can be permeable to at least one sample (substance) or to the substance mixtures and substantially impermeable for assay components, and at least for the targets. This separation layer can encompass the whole bottom of the assay cavity or just a part of it. If immobilization of the target is possible in a way that no diffusion of targets in the assay layer occurs while the assay is performed, the distinct assay cavities may be replaced by e.g. realisations in which the sample layer or the diffusion layer integrates the function of the assay cavities (figure 6D). Also these realisations of assay cavities may optionally be identically or differently coated to modify the wettability and the interaction of assay components as well as samples (substances) with the (functionally integrated) assay cavity (21).

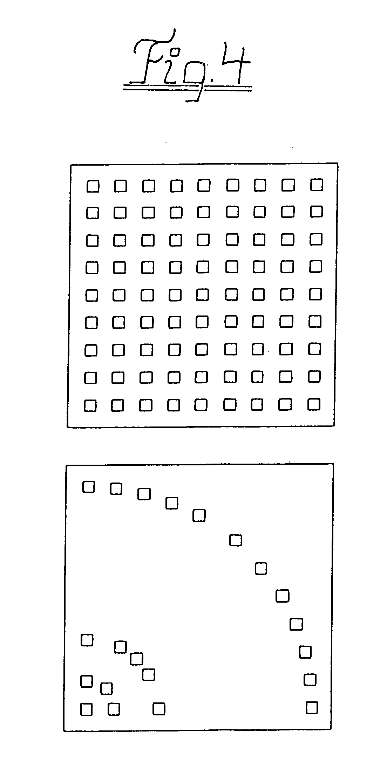

Figure 4 shows examples for the testing of different sample (substance) dilution-patterns. Depending on the arrangement of the discrete sites where immobilizing of the targets takes place/a testing is performed different dilution patterns can be probed. If for instance the discrete sites are equally/regularly spaced, linear dilution patterns are tested, as is illustrated in the top of figure 4. On the other hand logarithmic dilution patterns are tested if the discrete sites are logarithmically spaced, as is shown in the bottom of figure 4. Depending on the chosen assay, these different dilution patterns can be used to investigate slightest variations of relative and absolute concentrations of compound mixtures in e.g. the optimisation of cancer therapy or optimisation of reaction conditions in the solid phase organic chemistry.

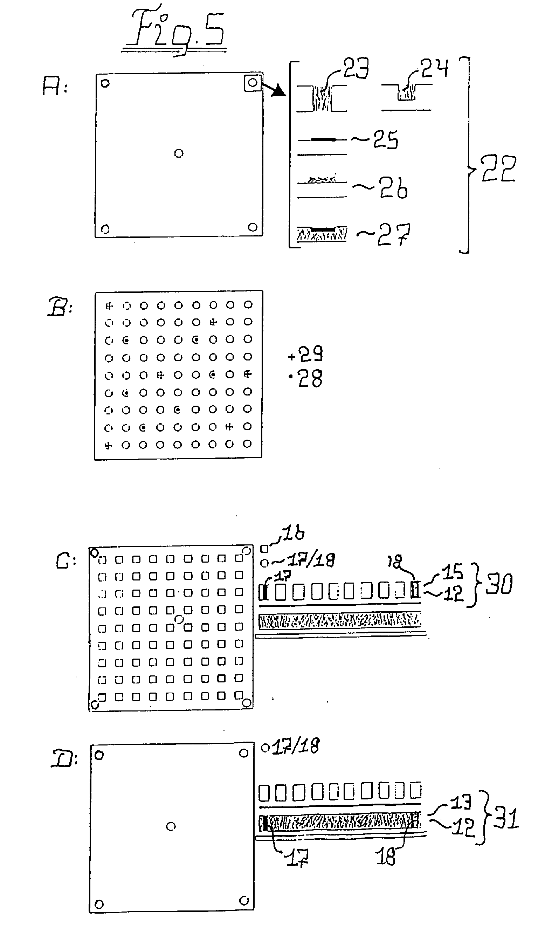

In figure 5 a few possible options for the design of sample layers are shown. Figure 5 A shows sample layers with several substance (sample) application sites (22). These substance application sites can be a fiber/resin-filled hole (23) or cavity (24) soaked with sample (substance) in a dry or wet form. It is also possible to spot the sample (substance) solution onto a solid support (25) or a fiber/resin pad on a solid support (26) and to dry it. Also a realisation is shown in which the sample (substance) is spotted onto a membrane with little (lateral) diffusion (27) followed by drying of said sample (substance) on the membrane. Alternatively, the sample layer can be connected to or substituted by a microfluidic device comprising ducts or channels connected to inlets (•; 28) and outlets (**†**; 29) in the diffusion layer as is shown in figure 5 B. It is also possible that the function of the sample layer is integrated into the assay layer (figure 5 C (30)) or into the diffusion layer (figure 5 D (31)), e.g. in form of separate micro cavities.

In figure 6 several options for the design of the diffusion layer are shown. In general the diffusion layer can be any layer capable of generating mixtures of substances by diffusion. The material has to obstruct convection and to minimize non-specific interaction of sample (substance) components. Functionally, the assay layer can be combined with the diffusion layer or the assay layer can consist out of a suited material to fulfill the function of the diffusion layer (figure 6 B, C, D). In the realisation shown in figure 6B, the boundary of diffusion layer and assay cavity is impermeable with respect to the target and permeable with respect to the substances and the same component of the device can be used as assay layer/sample application layer/diffusion layer (32). Another possible realisation for the combination of the assay layer, a sample application layer and the diffusion layer in one component of the device is shown in figure 6 C. In this realisation the targets are only present in distinct sites of the layer (33). Realisations are also possible in which the layer of figure 6C is completely functionalised with target (figure 6 D).

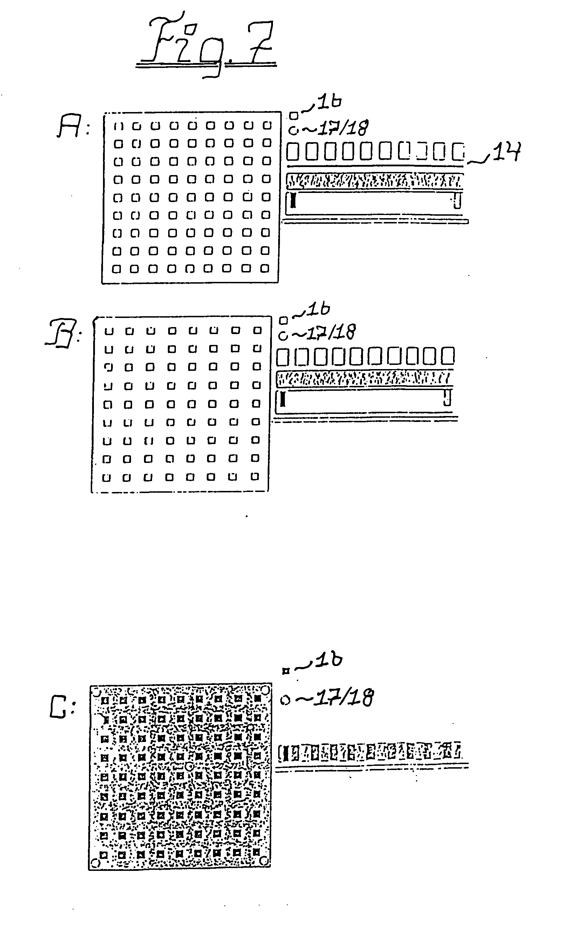

Figure 7 shows several options for the design of the separation medium. One possible realisation to separate assay and diffusion layer is the use of a separation layer (14), e.g. a semipermeable membrane (figure 7 A). In other realisations the separation layer can functionally be replaced by immobilizing the targets to the walls of the assay cavities or to supports (e.g. polystyrene beads) placed into the assay cavities that do not enter the diffusion layer (figure 7 B), or by immobilizing targets in a combined assay/diffusion layer as is shown in figure 7 C.

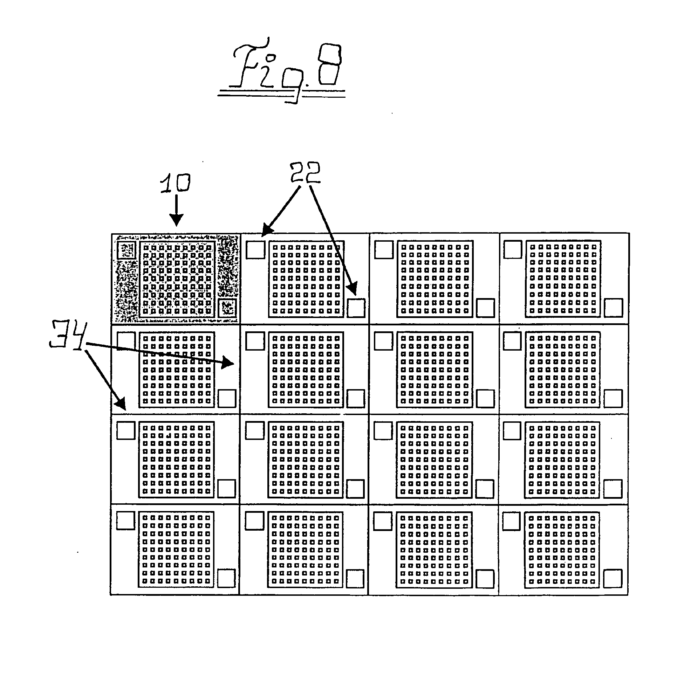

Figure 8 shows an example for the combination of 16 assay devices (10) used to test different combinations of samples (substances) with respect to one target, several targets with respect to one combination of samples (substances) or a combination thereof. In the realisation shown here, the samples (substances) are pipetted into the sample application sites (22), and the individual devices are separated from each other by diffusional barriers (34). The individual devices can be designed according to the designs described in figures 1 to 7.

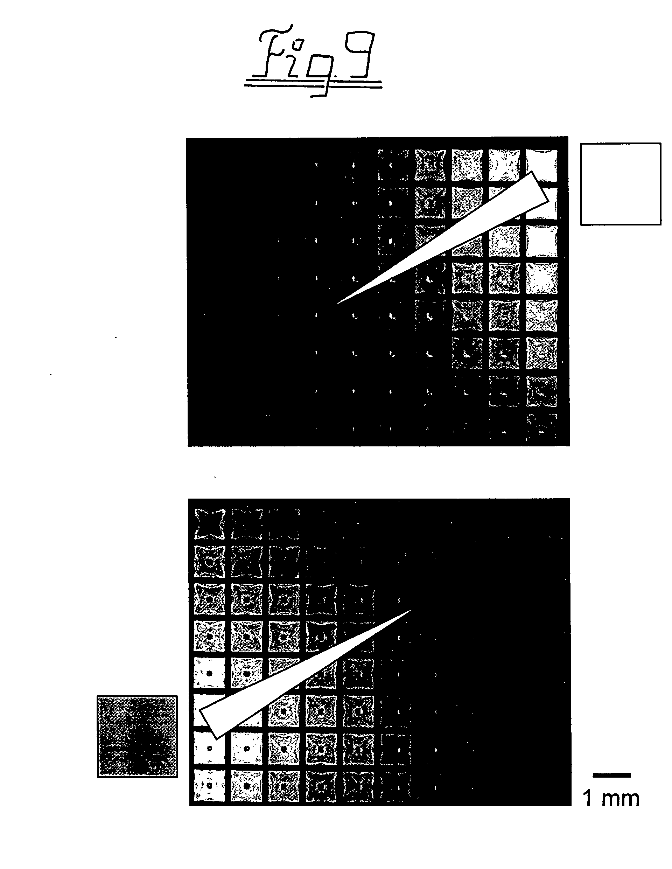

Figure 9 shows an experiment, in which an inverse diagonal substance gradient was established on a silicium nanotiter plate by diffusion. In this experiment nanotiterplates of 12 x 12 mm size were placed on an agarose bed as diffusion layer. Contact of the assay cavities with the diffusion layer was ensured by the presence of holes in the bottom of the assay cavities. Carboxy-tetramethylrhodamine and carboxy-fluorescein solutions were pipetted on 2 x 2 mm pieces of Whatman filter paper serving as sample application layer, dried and then placed onto the diffusion layer. The dimensions and positions of the samples are indicated. Images were recorded on a Zeiss Axioplan microscope (1.25 x lens) after 16 h incubation at 37 °C in a humid incubation chamber (Top: fluorescein fluorescence; bottom; rhodamine fluorescence).

When carboxy-tetramethylrhodamine (bottom: rhodamine fluorescence) and carboxy-fluorescein (top: fluorescein fluorescence) solutions were transferred to the diffusion layer from substance application sites an inverse diagonal gradient in the assay cavities of the silicium nanotiterplates was established as is shown in figure 9. This experiment clearly demonstrates, that the working principle of the novel diffusion assay can be used for the establishing of diagonal gradients, wherein on each position of the diffusion device substance mixtures with different concentrations are established.

In an assay, fluorescent molecules as shown here may serve as functionally inert reporter molecules for determining the concentrations of test substances at a specific position of the assay device.

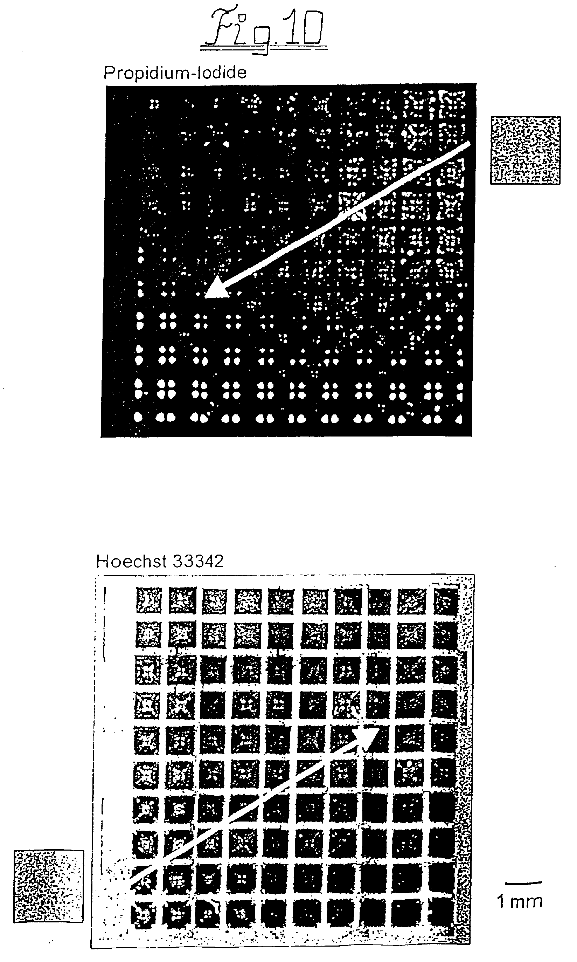

Figure 10 shows an example for assaying the joint interaction of two substances in a cellular assay. Adherent tissue culture cells serving as targets were seeded on a 12 x 12 mm nanotiter plate, placed in a 60 mm tissue culture dish. After two days of cultivation the nanotiter plate was placed on an agarose bed as diffusion layer. Contact of the assay cavities with the diffusion layer was given by holes in the bottom of the assay cavities. The adherent growth of the cells functionally replaced the separation layer. Two times 3 µl solutions of the DNA dyes Hoechst 33342 (0.1 mM) and propidium iodide (50 µg/ml) in water were applied on 2 x 2 mm pieces of Whatman filter paper serving as sample application layer. After each application of solution, the filter papers were dried. The dry filter papers were placed onto the agarose bed, where indicated. The square in the upper panel indicates the total size of the diffusion layer. Images were recorded on a Zeiss Axioplan microscope (5 x lens) after 16 h incubation at 37 °C in a humid incubation chamber (Top: propidium iodide fluorescence; bottom; same nanotiter plate but Hoechst 33342 fluorescence). The whole image was generated by combination of individual images in a mosaic-like fashion.

As is illustrated in figure 10, the Hoechst 33342 dye stains the nuclei of all cells, while propidium iodide only stains the nuclei of dead cells with a permeable plasma membrane. In the concentrations used, free propidium iodide has a faint fluorescence, while the Hoechst signal is only visible in the DNA-bound state. In the top panel it can be seen that only for a part of the gradient, does the test substance show activity, i. e. DNA staining. The bright corners of the nanotiter wells in the lower left of the propidium image are due to reflection at the meniscus of the cell culture medium. The bright appearance of the upper surface of the nanotiter plate in the Hoechst 33342 image was caused by the filter set used for detection of fluorescence and does not represent Hoechst 33342 fluorescence. This is apparent from the gradient-free homogeneity of this signal over the whole nanotiter plate.

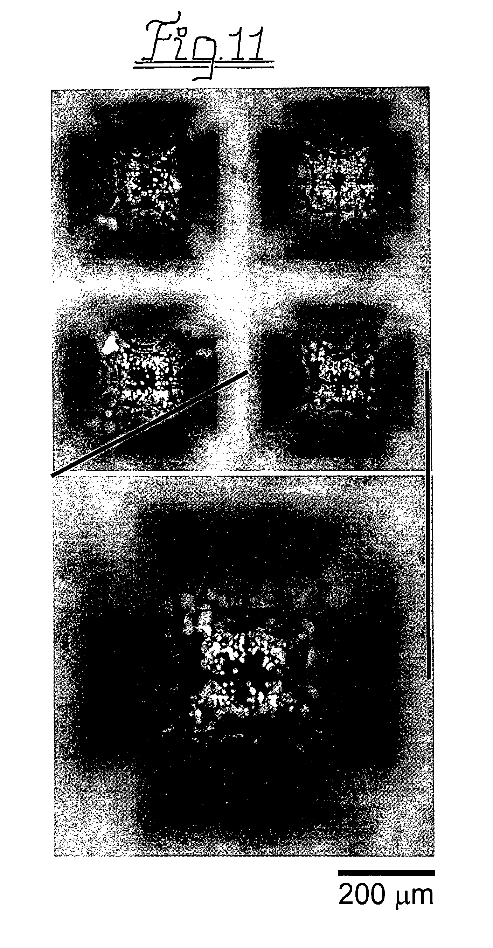

Figure 11 shows an enlargement of the four lower left assay cavities and a further enlargement of one of those four assay cavities as indicated in the figure 10. Each bright spot corresponds to one Hoechst 33342-stained cell nucleus.

## Claims

1. Device for assaying the interaction, especially the joint interaction, of at least two substances with at least one target, in which mixtures of said substances are generated, comprising a diffusion medium for generating mixtures of said substances by diffusion, and at least one discrete site for immobilizing at least one target with respect to the diffusion medium.

2. Device according to claim 1, **characterized in that** said diffusion medium is a diffusion layer.

3. Device according to claim 1 or claim 2, **characterized in that** diffusion in two dimensions, especially lateral diffusion, is decisive for the generation of said mixtures.

4. Device according to claim 1 or claim 2, **characterized in that** the diffusion medium is a three-dimensional diffusion medium and that the generation of said mixtures of said substances takes place in all three dimensions.

5. Device according to one of the preceding claims, **characterized in that** said discrete sites are part of or are formed by the diffusion medium.

6. Device according to one of claims 1 to 4, **characterized in that** said discrete sites are part of or are formed by an assay medium, especially an assay layer.

7. Device according to claim 6, **characterized in that** the assay medium is reversibly connected with the device, especially the diffusion medium.

8. Device according to one of the preceding claims, **characterized in that** said discrete sites are in the form of cavities, preferably formed in said assay layer.

9. Device according to one of the preceding claims, **characterized in that** said discrete sites are arranged in a preferably regular pattern, wherein preferably said discrete sites are equally or logarithmically spaced for probing linear or logarithmic dilution patterns with respect to the concentration gradients of substances in the diffusion medium.

10. Device according to one of the preceding claims, **characterized in that** said discrete sites are separated from the diffusion medium, especially by a separation layer.

11. Device according to claim 10, **characterized in that** said separation layer is formed by a semipermeable membrane, wherein preferably said semipermeable membrane is permeable to at least one substance of the substance mixture and substantially impermeable to said targets.

12. Device according to claim 11, **characterized in that** said semipermeable membrane is functionally replaced by covalent or non-covalent coupling of said targets to an assay medium in which said discrete sites are preferably formed as cavities, or by covalent or non-covalent coupling of said targets to microspheres located inside said cavities.

13. Device according to one of the preceding claims, further comprising at least one substance application site.

14. Device according to claim 13, **characterized in that** said substance application sites are part of or are formed by a sample medium, especially a sample application layer.

15. Device according to claim 13 or claim 14, **characterized in that** said substance application sites are in the form of a fiber/resin-filled hole, a fiber/resin pad, a cavity, a dried substance spot on a solid support and/or a membrane with little lateral diffusion.

16. Device according to claim 14 or claim 15, **characterized in that** the sample medium is connected to or substituted by a microfluidic device comprising ducts or channels connected to inlets and outlets in the diffusion medium.

17. Device according to one of the preceding claims, further comprising a solid support.

18. Device according to one of the preceding claims, comprising the following sequence of components:
- a solid support,
- a sample application layer, comprising substance application sites,
- a diffusion layer,
- a separation layer, and
- an assay layer, comprising discrete target immobilizing sites.

19. Device according to one of the preceding claims, **characterized in that** sample application layer and assay layer are integrated into each other.

20. Device according to one of the preceding claims, **characterized in that** sample application layer and diffusion layer are integrated into each other.

21. Assay device comprising at least two of the devices according to one of the preceding claims.

22. Method for assaying the interaction, especially the joint interaction, of at least two substances with at least one target, in which mixtures of said substances are generated, **characterized in that** said mixtures are generated by diffusion of said substances in a diffusion medium, especially a diffusion layer, in a way that the concentrations of at least two substances vary at any place where a testing is performed, said mixtures interact with targets substantially immobilized with respect to the diffusion medium at discrete sites, and said interaction is detected, and the concentrations of the substances are derived from the positions of the device where said interaction is detected.

23. Method according to claim 22, **characterized in that** at least one of said substances does not directly interact with said target but influences the interaction of at least one other substance interacting with said target.

24. Method according to claim 22 or 23, performed on a device **characterized by** at least one feature as defined in at least one of claims 2 to 21.

25. Method according to one of claims 22 to 24, **characterized in that** the interaction of said substances with said target is preferably read-out by fluorescence, mass spectrometry, high performance liquid chromatography, reverse PCR, capillary electrophoresis and/or by changes of the electric properties of any assay constituent, preferably addressed by at least one microelectrode.

26. Method according to one of claims 22 to 25 and use of the device according to claims 1 to 21, **characterized in that** said targets are prokaryotes, eukaryotic cells, cellular organelles, biomolecules from natural or synthetic source and/or synthetic molecules with no correspondence in nature.

27. Method and use according to claim 26, for testing of combinations of anticancer drugs for optimisation of these combinations in anticancer therapy or for testing of combinations of antibiotics.

28. Use of a device according to one of claims 1 to 21 or use of claim 26 or 27, **characterized in that** at least two of said devices are used for testing different combinations of substances in relation to the same target.

29. Use of a device according to one of claims 1 to 21 or use of claim 26 or 27, **characterized in that** at least two of said devices are used for testing different targets in relation to one combination of substances.

30. Use of claim 28 or 29, **characterized in that** said device is used to supply data for establishing models describing the behaviour of a target in response to the combinations of substances in a predictive fashion.
